# EUROPEAN PATENT APPLICATION

(11) **EP 0 873 753 A1**
(43) Date of publication of application: **28.10.1998**
(21) Application number: 98302747.5
(22) Date of filing: 08.04.1998
(51) Int. Cl.: A61K 31/435, A61K 31/445

(54) **Use of NK-1 receptor antagonists for the manufacture of a medicament in the treatment of symptoms of irritable bowel syndrome**

(30) Priority: 23.04.1997 US 44250 P
(71) Applicant: PFIZER INC., New York, N.Y. 10017 (US)
(72) Inventor: Williams, Stephen Alaric, North Stonington, Connecticut 06359 (US)
(74) Representative: Hayles, James Richard

(57) **Abstract**

The invention relates to the use of a NK-1 receptor antagonist, in particular a substance P receptor antagonist, for the manufacture of a medicament for the treatment of symptoms of irritable bowel syndrome.

## Description

### Background of the Invention

The present invention relates to a method of treating or preventing the symptoms of irritable bowel syndrome, in particular abdominal pain associated with irritable bowel syndrome, in mammals, including humans, using an NK-1 receptor antagonist. The present invention also relates to a method of preventing or treating symptoms of irritable bowel syndrome, in particular abdominal pain associated with irritable bowel syndrome, in mammals, including humans, using certain quinuclidine derivatives, piperidine derivatives and related compounds that are substance P receptor antagonists.

The following references refer, collectively, to quinuclidine, piperidine, ethylene diamine, pyrrolidine and azanorbomane derivatives and related compounds that exhibit activity as substance P receptor antagonists: United States Patent 3,560,510 (issued February 2, 1991); United States Patent 4,358,446 (issued November 9, 1982); United States Patent 4,680,283 (issued July 14, 1987); United States Patent 4,552,960 (issued November 12, 1985); United States Patent 4,767,759 (issued August 30, 1988); United States Patent 5,138,060 (issued August 11, 1992); United States Patents 5,162,339 (issued November 10, 1992); United States Patent 5,232,929 (issued August 3, 1993); United States Patent 5,364,943 (issued November 15, 1994); United States Patent 5,451,586 (issued September 19, 1995); United States Patent 5,498,614 (issued March 12, 1996); United States Patent 5,332,817 (issued July 26, 1994); United States Patent 5,422,354 (issued June 6, 1995); United States Patent 5,216,163 (issued June 1, 1993); United States Patent 5,442,068 (issued August 15, 1995); United States Patent 5,373,003 (issued December 13,1994); United States Patent 5,294,744 (issued March 15, 1993); United States Patent 5,393,762 (issued February 28, 1995); United States Patent 5,340,826 (issued August 23, 1994); United States Patent 5,360,820 (issued November 1, 1994); United States patent application serial number 08/060,195 (filed May 10, 1993); United States patent application serial number 08/377,552 (filed January 24, 1995); European patent application 436,334 (published July 10, 1991); European patent application 499,313 (published February 4, 1992); European patent application 520,555 (published June 17, 1992); European patent application 522,808 (published July 3, 1992); European patent published 653,208 (published May 31, 1995); European patent application 655,246 (published May 31, 1995); European patent application 100,158 (published February 8, 1984); European patent application 15,628 (published September 17, 1980); PCT patent application WO 91/09844 (published July 11, 1991); PCT patent application WO 93/01170 (published January 21, 1993); PCT patent application WO 92/21677 (published December 10, 1992); PCT patent application WO 92/17449 (published October 15, 1992); PCT patent application WO 94/26740 (published November 24, 1994); PCT patent application WO 90/05525 (published May 31, 1990); PCT patent application WO 90/05729 (published May 31, 1990); PCT patent application WO 91/18899 (published December 12, 1991); PCT patent application WO 94/11368 (published May 26, 1994); PCT patent application WO 93/06099 (published April 1, 1993); PCT patent application WO 93/10073 (published May 27, 1993); PCT patent application WO 93/23380 (published November 25, 1995); PCT patent application WO 94/03445 (published February 17, 1994); PCT patent application WO 93/19064 (published September 30, 1993); PCT patent application WO 92/20676 (published November 26, 1992); PCT patent application WO 94/13663 (published June 23, 1994); PCT patent application WO 94/10170 (published May 11, 1994); PCT patent application WO 94/08997 (published April 28, 1994); PCT patent application WO 94/20500 (published September 15, 1994); PCT patent application WO 95/07908 (published March 23, 1995); PCT patent application WO 94/04496 (published March 3, 1994); PCT patent application WO 95/02595 (published January 26, 1995); PCT patent application WO 95/07886 (published March 23, 1995); PCT patent application WO 95/18129 (published July 6, 1995); PCT patent application WO 92/01688 (published February 6, 1992); PCT patent application WO 92/12151 (published July 23, 1992); PCT patent application WO 92/15585 (published September 17, 1992); PCT patent application WO 93/00331 (published January 7, 1993); PCT patent application WO 93/00330 (published January 7, 1993); PCT patent application WO 92/06079 (published April 16, 1992); PCT patent application WO 93/14084 (published July 22, 1993); PCT patent application WO 93/01169 (published January 21, 1993); PCT patent application WO 93/01159 (published January 21, 1993); PCT patent application WO 92/20661 (published November 26, 1992); PCT patent application WO 97/08144 (published March 6, 1997); European patent application 528,495 (published February 24, 1993); European patent application 517,589 (published December 12, 1992); European patent application 428,434 (published May 22, 1991); and European patent application 360,390 (published March 28, 1990). All of the foregoing PCT patent applications designate the United States. Each of the foregoing United States, European and PCT patents and patent applications is incorporated herein by reference in its entirety. Each PCT patent application referred to above designates the United States. In addition to the NK-1 receptor antagonists and substance P receptor antagonists specifically referred to herein, the NK-1 receptor antagonists and substance P receptor antagonists referred to in the foregoing patents and patent applications may be used in accord with the methods of the present invention.

### Summary of the Invention

This invention relates to a method of treating or preventing symptoms of irritable bowel syndrome, in particular pain associated with irritable bowel syndrome, in a mammal, including a human, comprising administering to such mammal an amount of an NK-1 receptor antagonist that is effective in treating or preventing such symptoms.

This invention also relates to a method of treating or preventing symptoms of imitable bowel syndrome, in particular abdominal pain associated with irritable bowel syndrome, in a mammal, including a human, comprising administering to such mammal an amount of a substance P receptor antagonist that is effective in treating or preventing such symptoms.

This invention also relates to a method of treating or preventing symptoms of irritable bowel syndrome, in particular abdominal pain associated with irritable bowel syndrome, in a mammal comprising administering to said mammal
(A) an amount of a compound of the formula 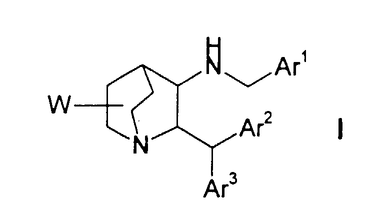 wherein
   W is Y or X(CH₂)ₙ- wherein n is an integer ranging from 0 to 4;
   Y is hydrogen, (C₁-C₆)alkyl, (C₂-C₆)alkenyl or (C₃-C₈)cycloalkyl wherein each of the foregoing alkyl, alkenyl, and cycloalkyl Y groups is optionally substituted by 1 to 3 R⁴ groups;
   X is hydroxy, (C₁-C₆)alkoxy, -C(O)NR¹R², -CO₂R¹, -CHR¹OR², -CHR¹NR²R³, -C(O)R¹, -C(O)NR¹OR² or aryl, wherein said aryl is selected from phenyl, naphthyl, pyridyl, quinolyl, thienyl, furyl, phenoxyphenyl, oxazolyl, tetrazolyl, thazolyl, imidazolyl and pyrazolyl, and wherein said aryl and alkoxy groups are optionally substituted by 1 to 3 R⁴ groups;
   Ar¹, Ar² and Ar³ are each independently selected from phenyl, naphthyl, pyridyl, quinolyl, thienyl, furyl, phenoxyphenyl , oxazolyl, tetrazolyl, thiazolyl, imidazolyl and pyrazolyl, wherein the foregoing Ar² and Ar³ groups are optionally substituted by 1 to 3 R⁶ groups, and the foregoing Ar¹ group is optionally substituted by 1 to 3 R⁵ groups;
   R¹, R² and R³ are each independently selected from hydrogen, (C₁-C₆)alkyl, (C₁-C₆)alkoxy, (C₃-C₈)cycloalkyl, aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl; and heterocyclyl, wherein said heterocyclyl is selected from pyrrolidino, piperidino, morpholino, piperazinyl and thiamorpholino, wherein said aryl and heterocyclyl groups are optionally substituted by 1 to 3 R⁴ groups;
   each R⁴ is independently selected from halo, nitro, amino, (C₁-C₄)alkyl, (C₁-C₄)alkoxy, trifluoromethyl and trifluoromethoxy;
   each R⁵ is independently selected from halo, (C₁-C₆)alkyl optionally substituted with from one to three halo groups, (C₁-C₆)alkoxy optionally substituted with from one to three halo groups, (C₁-C₆)alkylsulfinyl, (C₂-C₆)alkenyl, (C₁-C₆)alkylthio, (C₁-C₆)alkylsulfonyl, (C₁-C₆)alkylsulfonylamino, and di-(C₁-C₆)alkylamino wherein said alkyl groups and the alkyl moieties of said R⁵ groups are optionally substituted by (C₁-C₆)alkylsulfonyl or (C₁-C₆)alkylsufinyl;
   and each R⁶ is independently selected from (C₁-C₆)alkylamino, trifluoromethyl and trifluoromethoxy;
   or a pharmaceutically acceptable salt of such compound, that is effective in treating or preventing symptoms of, in particular abdominal pain associated with, irritable bowel syndrome; or
(B) an amount of a compound of the formula 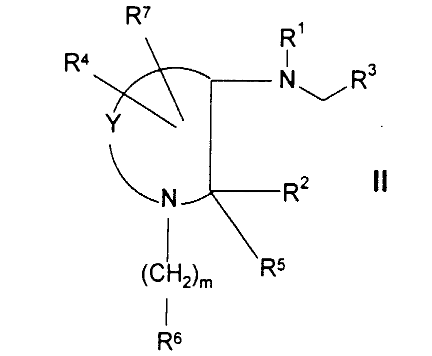
   wherein Y is (CH₂)ₙ where n is an integer ranging from 1 to 6, and any one of the carbon-carbon single bonds in said (CH₂)ₙ may optionally be replaced by a carbon-carbon double bond, and any one of the carbon atoms of said (CH₂)ₙ may optionally be substituted with R⁴ or R⁷;
   m is an integer ranging from 0 to 8, and any one of the carbon-carbon single bonds of said (CH₂)ₘ may optionally be replaced by a carbon-carbon double bond or a carbon-carbon triple bond;
   R¹ is hydrogen or (C₁-C₈)alkyl optionally substituted with hydroxy, alkoxy or fluoro;
   R² is selected from hydrogen, (C₁-C₆)alkyl, (C₃-C₇)cycloalkyl wherein one of the carbon atoms of said cycloalkyl may optionally be replaced by nitrogen, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl-(C₂-C₆)alkyl, benzhydryl and benzyl, wherein each of said aryl and heteroaryl groups and the phenyl moieties of said benzyl, phenyl-(C₂-C₆)alkyl and benzhydryl may optionally be substituted with 1 to 3 substituents independently selected from halo, nitro, (C₁-C₆)alkyl, (C₁-C₆)alkoxy, trifluoromethyl, amino, (C₁-C₆)alkylamino, (C₁-C₆)alkyl-C(O)-O-, (C₁-C₆)alkyl-O-C(O)-, (C₁-C₆)alkyl, (C₁-C₆)alkyl-C(O)-O-, (C₁-C₆)alkyl-C(O)-(C₁-C₆)alkyl-O-, (C₁-C₆)alkyl-C(O)-, (C₁-C₆)alkyl-C(O)-(C₁-C₆)alkyl-, di-(C₁-C₆)alkylamino, -C(O)-NH-(C₁-C₆)alkyl, (C₁-C₆)alkyl-C(O)-NH-(C₁-C₆)alkyl, -NHC(O)H and -NHC(O)-(C₁-C₆)alkyl; and wherein one of the phenyl moieties of said benzhydryl may optionally be replaced by naphthyl, thienyl, furyl or pyridyl;
   R⁵ is hydrogen, phenyl or (C₁-C₆)alkyl;
   or R² and R⁵, together with the carbon to which they are attached, form a saturated carbocyclic ring having from 3 to 7 carbon atoms wherein one of said carbon atoms may optionally be replaced by oxygen, nitrogen or sulfur;
   R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of said carbon atoms may optionally be replaced by nitrogen, oxygen or sulfur; wherein each of said aryl and heteroaryl groups may optionally be substituted with 1 to 3 substituents, and said (C₃-C₇)cycloalkyl may optionally be substituted with one or two substituents, each of said substituents being independently selected from halo, nitro, (C₁-C₆)alkyl, (C₁-C₆)alkoxy, methyl, trifluoromethyl, trifluoromethoxy, phenyl, amino, (C₁-C₆)alkylamino, -C(O)-NH-(C₁-C₆)alkyl, (C₁-C₆)alkyl-C(O)-NH-(C₁-C₆)alkyl, -NHC(O)H and -NHC(O)-(C₁-C₆)alkyl; and
   R⁴ and R⁷ are each independently selected from hydrogen, hydroxy, halo, amino, oxo (=O), nitrile, (C₁-C₆)alkylamino, di-(C₁-C₆)alkylamino, (C₁-C₆)alkoxy, (C₁-C₆)alkyl-O-C(O)-, (C₁-C₆)alkyl-O-C(O)-(C₁-C₆)alkyl-, hydroxy-(C₁-C₆)alkyl, (C₁-C₆)alkoxy-(C₁-C₆)alkyl, (C₁-C₆)alkyl-C(O)-O-, (C₁-C₆)alkyl-C(O)-(C₁-C₆)alkyl-O-, (C₁-C₆)alkyl-C(O)-, (C₁-C₆)alkyl-C(O)-(C₁-C₆)alkyl-, and the radicals set forth in the definition of R²;
   R⁶ is hydrogen, NHC(O)R⁸, NHCH₂R⁸, SO₂R⁸ or one of the radicals set forth in the definitions of R², R⁴ and R⁷; and,
   R⁸ is (C₁-C₆)alkyl, hydrogen, phenyl, or phenyl-(C₁-C₆)alkyl;
   or a pharmaceutically acceptable salt of such compound, that is effective in treating or preventing symptoms of, in particular abdominal pain associated with, irritable bowel syndrome; or
(C) an amount of a compound of the formula 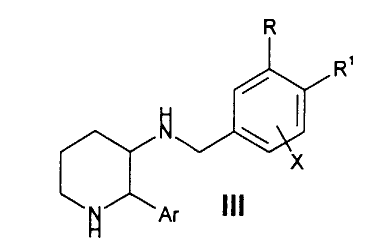 wherein
   R is C₁-C₈ alkoxy, C₁-C₈ alkyl substituted by halo, C₂-C₈ alkenyl substituted by halo, C₂-C₈ alkynyl substituted by halo, or C₁-C₈ alkyl substituted by halo and hydroxy;
   R¹ is H, halo, or C₁-C₈ alkoxy;
   or R and R¹ are taken together with the two carbons to which they are attached to form a fused C₄-C₆ cycloalkyl ring wherein one carbon atom is optionally replaced by oxygen and wherein one or two carbon carbon atoms are optionally substituted by 1 to 5 substituents independently selected from halo, C₁-C₆ alkyl and C₁-C₆ alkyl substituted by halo;
   X is C₁-C₆ alkoxy, C₁-C₆ alkoxy substituted by halo, phenoxy or halo; and
   Ar is phenyl optionally substituted by halo;
   or a pharmaceutically acceptable salt of such compound, that is effective in treating or preventing symptoms of, in particular abdominal pain associated with, irritable bowel syndrome.

In a preferred embodiment, the compound that is administered in accord with the present invention is selected from the group consisting of
(2S,3S)-2-diphenylmethyl-3-(5-tert-butyl-2-methoxybenzyl)amino-1-azabicyclo[2.2.2]octane,
(2S,3S)-3-(2-methoxybenzyl)amino-2-phenyl-piperidine,
(2S,3S)-3-(5-trifluoromethoxy-2-methoxybenzyl)amino-2-phenyl-piperidine,
(2S,3S)-2-diphenylmethyl-3-(5-isopropyl-2-methoxybenzyl)amino-1-azabicyclo[2.2.2]octane,
(2S,3S)-2-diphenylmethyl-3-(2-methoxybenzyl)amino-1 -azabicyclo[2.2.2]octane,
(3R,4S,5S,6S)-5-(5-isopropyl-2-methoxybenzyl)amino-6-diphenylmethyl-1-azabicyclo[2.2.2]octane-3-carboxylic acid,
and the pharmaceutically acceptable salts of the foregoing compounds.

In another preferred embodiment, the compound that is administered in accord with the present invention is (2S,3S)-2-diphenylmethyl-3-(5-tert-butyl-2-methoxybenzyl)amino-1-azabicyclo[2.2.2]octane or a pharmaceutically accetable salt of said compound.

In general, subjects suffering from imitable bowel syndrome have, or express, the following symptoms: (a) abdominal pain or discomfort that is relieved with defecation or is associated with a change in frequency or consistency of stools; and (b) an irregular pattem of defecation at least 25% of the time in which 3 or more of the following criteria are met: (I) stool frequency is altered, (ii) stool form is altered (hard or loose and watery), (iii) stool passage is aitered (strained or urgent passage, or feeling of incomplete evacuation), (iv) passage of mucus, and (v) bloated feeling or feeling of abdominal distension. Symptoms associated with irritable bowel syndrome are further described in Drossman et al., Gastroenterol. Int., vol. 3, pages 159-172 (1990).

The term "halo", as used herein, unless otherwise indicated, includes chloro, fluoro, bromo and iodo.

The term "alkyl", as used herein, unless otherwise indicated, includes saturated monovalent hydrocarbon radicals having straight cyclic or branched moieties, or combinations thereof. It is understood that for cyclic moieties at least three carbon atoms are required in said alkyl group.

The term "alkenyl", as used herein, unless otherwise indicated, refers to alkyl radicals, wherein alkyl is as defined above, having one carbon-carbon double bond including, but not limited to, ethenyl, 1- and 2-propenyl, 2-methyl-1-propenyl, and 1- and 2-butenyl.

The term "alkoxy", as used herein, unless otherwise indicated, refers to an -O-alkyl radical, wherein alkyl is as defined above, including, but not limited to, methoxy, ethoxy, propoxy, isopropoxy, n-butoxy, isobutoxy and t-butoxy.

The term "alkylthio", as used herein, unless otherwise indicated, refers to an -S-alkyl radical, wherein alkyl is as defined above, including, but not limited to, methylthio, ethylthio, n-propylthio, isopropylthio, n-butylthio, isobutylthio, and t-butylthio.

The phrase "pharmaceutically acceptable salt(s)", as used herein, unless otherwise indicated, includes salts of acidic or basic groups which may be present in the compounds of formulas I, II and III. The compounds of formulas I, II and III that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts.

Those compounds of formulas I, II and III that are acidic in nature are capable of forming base salts with various pharmacologically acceptable cations. Examples of such salts include the alkali metal or alkaline earth metal salts and, particularly, the calcium, magnesium, sodium and potassium salts of the compounds of formulas I, II and III.

Certain compounds of formulas I, II and III may have asymmetric centers and therefore exist in different enantiomeric and diastereomic forms. This invention relates to the use of all optical isomers and stereoisomers of the compounds of formulas I, II and III, and mixtures thereof.

The present invention includes the use of the compounds of formulas I and II, and the pharmaceutically acceptable salts thereof, wherein one or more hydrogen, carbon or other atoms are replaced by isotopes thereof. The use of such compounds in accord with the present invention may be useful in research and pharmacokinetic studies.

### Detailed Description of the Invention

The compounds of formulas I, II and III may be prepared according to methods familiar to those skilled in the art. Unless otherwise indicated, the structural formulas I, II and III are defined as above.

Compounds of formula I may be prepared as described in PCT patent application WO 92/20676 (published November 26, 1992), PCT patent application WO 90/05729 (published May 31, 1990), PCT patent application WO 94/11368 (published May 26, 1994), PCT patent application WO 92/21677 (published December 10, 1992), and United States patent application serial number 08/377,552 (filed January 24, 1995). The foregoing United States patent application and PCT patent applications (each of which designates the United States) are incorporated herein by reference in their entirety.

Compounds of formula II may be prepared as described in PCT patent application WO 93/00331 (published January 7, 1993) and PCT patent application WO 93/01170 (published January 21, 1993). The foregoing PCT applications, which designate the United States, are incorporated herein by reference in its entirety.

Compounds of formula III may be prepared as described in PCT patent application WO 97/08144 (published March 6, 1997).

The compounds of formulas I, II and III may have asymmetric carbon atoms and therefore exist in different enantiomeric and diastereomic forms. Diasteromeric mixtures can be separated into their individual diastereomers on the basis of their physical chemical differences by methods known to those skilled in the art, for example, by chromatography or fractional crystallization. Enantiomers may be separated by converting the enantiomeric mixtures into a diastereomeric mixture by reaction with an appropriate optically active compound (e.g., alcohol), separating the diastereomers and converting (e.g., hydrolyzing) the individual diastereomers to the corresponding pure enantiomers. All such isomers, including diastereomer mixtures and pure enantiomers, may be used in accord with, and are considered to be part of, the present invention.

The compounds of formulas I, II and III that are basic in nature are capable of forming a wide variety of different salts with various inorganic and organic acids, including amino acids. Although such salts must be pharmaceutically acceptable for administration to mammals, it is often desirable in practice to initially isolate the compound of formula I or II from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired acid salt can also be precipitated from a solution of the free base in an organic solvent by adding to the solution an appropriate mineral or organic acid.

Those compounds of formulas I, II and III that are acidic in nature are capable of forming base salts with various pharmacologically acceptable cations. Examples of such salts include the alkali metal or alkaline-earth metal salts and particularly the sodium and potassium salts. These salts are all prepared by conventional techniques. The chemical bases which are used as reagents to prepare the pharmaceutically acceptable base salts of this invention are those which form non-toxic base salts with the acidic compounds of formulas I and II. Such non-toxic base salts include those derived from such pharmacologically acceptable cations as sodium, potassium, calcium, magnesium, various amine cations, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The compounds of formulas I, II and III and the pharmaceutically acceptable salts thereof (hereinafter referred to, collectively, as the "therapeutic agents") are useful as substance P receptor antagonists, i.e., they possess the ability to antagonize the effects of tachykinins at the substance P receptor site in mammals. They and other NK-1 receptor antagonists are able to function as therapeutic agents in the treatment or prevention of the symptoms of irritable bowel syndrome, in particular pain associated with irritable bowel syndrome, in mammals, including humans. Other substance P receptor antagonists that are expected to exhibit activity for the treatment or prevention of the symptoms of irritable bowel syndrome, in particular pain associated with irritable bowel syndrome, in mammals are referred to above in the "Background of the Invention".

The therapeutic agents referred to above and other NK-1receptor antagonists can be administered via oral, rectal, topical and parenteral routes. In general, these compounds are most desirably administered in dosages ranging from about 5.0 mg up to about 1500 mg per day, although variations will necessarily occur depending upon the weight and condition of the subject being treated and the particular route of administration chosen. However, a dosage level that is in the range of about 0.001 mg to about 21 mg per kg of body weight per day is most desirably employed. The preferred dosage for oral administration is from about 0.001 to about 5 mg per kg of body weight per day. Ointments or eyedrops will preferably contain the active agent in a concentration of about 0.01 to about 5 percent, more preferably about 1%.

Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The therapeutic agents referred to above and other NK-1 receptor antagonists may be administered alone or in combination with pharmaceutically acceptable carriers or diluents by any of the routes previously indicated, and such administration may be carried out in single or multiple doses. More particularly, the therapeutic agents may be administered in a wide variety of different dosage forms, i.e., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic agents are present in such dosage forms at concentration levels ranging from about 5.0% to about 70% by weight

For oral administration, tablets incorporating the therapeutic agents may also contain various excipients such as microcrystalline cellulose, sodium citrate, calcium carbonate, dicalcium phosphate and glycine may be employed along with various disintegrants such as starch (and preferably com, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the therapeutic agents may be combined with various sweetening or flavoring agents, coloring matter or dyes, and, if so desired, emulsifying and/or suspending agents as well, together with such diluents as water, ethanol, propylene glycol, glycerin and various combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art. For rectal administration, the therapeutic agents may be incorporated in a suppository composition.

The activity of the therapeutic agents referred to above as substance P receptor antagonists may be determined by their ability to inhibit the binding of substance P at its receptor sites in bovine caudate tissue, employing radioactive ligands to visualize the tachykinin receptors by means of autoradiography. The substance P antagonizing activity of the herein described compounds may be evaluated by using the standard assay procedure described by M. A. Cascieri et al., as reported in the Journal of Biological Chemistry, Vol. 258, p. 5158 (1983). This method essentially involves determining the concentration of the individual compound required to reduce by 50% the amount of radiolabelled substance P ligands at their receptor sites in said isolated cow tissues, thereby affording characteristic IC₅₀ values for each compound tested.

In this procedure, bovine caudate tissue is removed from a -70°C freezer and homogenized in 50 volumes (w./v.) of an ice-cold 50 mM Tris (i.e., trimethamine which is 2-amino-2-hydroxymethyl-1,3-propanediol) hydrochloride buffer having a pH of 7.7. The homogenate is centrifuged at 30,000 x G for a period of 20 minutes. The pellet is resuspended in 50 volumes of Tris buffer, rehomogenized and then recentrifuged at 30,000 x G for another twenty-minute period. The pellet is then resuspended in 40 volumes of ice-cold 50 mM Tris buffer (pH 7.7) containing 2 mM of calcium chloride, 2 mM of magnesium chloride, 4 µg/ml of bacitracin, 4µg/ml of leupeptin, 2µg of chymostatin and 200 µg/ml of bovine serum albumin. This step completes the production of the tissue preparation.

The radioligand binding procedure is then carried out in the following manner, viz., by initiating the reaction via the addition of 100 µl of the test compound made up to a concentration of 1 µM, followed by the addition of 100 µl of radioactive ligand made up to a final concentration 0.5 mM and then finally by the addition of 800 µl of the tissue preparation produced as described above. The final volume is thus 1.0 ml, and the reaction mixture is next vortexed and incubated at room temperature (ca. 20°C) for a period of 20 minutes. The tubes are then filtered using a cell harvester, and the glass fiber filters (Whatman GF/B) are washed four times with 50 mM of Tris buffer (pH 7.7), with the filters having previously been presoaked for a period of two hours prior to the filtering procedure. Radioactivity is then determined in a Beta counter at 53% counting efficiency, and the IC₅₀ values are calculated by using standard statistical methods.

## Claims

1. The use
(A) of a compound of the formula 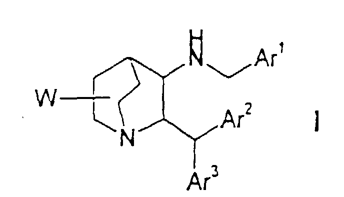 wherein
W is Y or X(CH₂)ₙ- wherein n is an integer ranging from 0 to 4;
Y is hydrogen, (C₁-C₆)alkyl, (C₂-C₆)alkenyl or (C₃-C₈)cycloalkyl wherein each of the foregoing alkyl, alkenyl, and cycloalkyl Y groups is optionally substituted by 1 to 3 R⁴ groups;
X is hydroxy, (C₁-C₆)alkoxy, -C(O)NR¹R², -CO₂R¹, -CHR¹OR², -CHR¹NR²R³, -C(O)R¹ -C(O)NR¹OR² or aryl, wherein said aryl is selected from phenyl, naphthyl, pyridyl, quinolyl, thienyl, furyl, phenoxyphenyl, oxazolyl, tetrazolyl, thazolyl, imidazolyl and pyrazolyl, and wherein said aryl and alkoxy groups are optionally substituted by 1 to 3 R⁴ groups;
Ar¹, Ar² and Ar³ are each independently selected from phenyl, naphthyl, pyridyl, quinolyl, thienyl, furyl, phenoxyphenyl, oxazolyl, tetrazolyl, thiazolyl, imidazolyl and pyrazolyl, wherein the foregoing Ar² and Ar³ groups are optionally substituted by 1 to 3 R⁶ groups, and the foregoing Ar¹ group is optionally substituted by 1 to 3 R⁶ groups;
R¹, R² and R³ are each independently selected from hydrogen, (C₁-C₅)alkyl, (C₁-C₆)alkoxy, (C₃-C₈)cycloalkyl, aryl, wherein said aryl is selected from phenyl, naphthyl, pyridyl, quinolyl, thienyl, furyl, phenoxyphenyl, oxazolyl, tetrazolyl, thiazolyl, imidazolyl and pyrazolyl ; and heterocyclyl, wherein said heterocyclyl is selected from pyrrolidino, piperidino, morpholino, piperazinyl and thiamorpholino, wherein said aryl and heterocyclyl groups are optionally substituted by 1 to 3 R⁴ groups.
each R⁴ is independently selected from halo, nitro, amino, (C₁-C₄)alkyl, (C₁-C₄)alkoxy, trifluoromethyl and trifluoromethoxy ;
each R⁵ is independently selected from halo, (C₁-C₆)alkyl optionally substituted with from one to three halo groups. (C₁-C₆)alkoxy optionally substituted with from one to three halo groups. (C₁-C₆)alkylsulfinyl, (C₂-C₆)alkenyl, (C₁-C₆)alkylthio, (C₁-C₆)alkylsulfonyl, (C₁-C₆)alkylsulfonylamino, and di-(C₁-C₆)alkylamino wherein said alkyl groups and the alkyl moieties of said R⁶ groups are optionally substituted by (C₁-C₆)alkylsulfonyl or (C₁-C₆)alkylsulfinyl ;
and each R⁶ is independently selected from (C₁-C₆)alkylamino, trifluoromethyl and trifluoromethoxy;
or a pharmaceutically acceptable salt of such compound; or
(B) of a compound of the formula 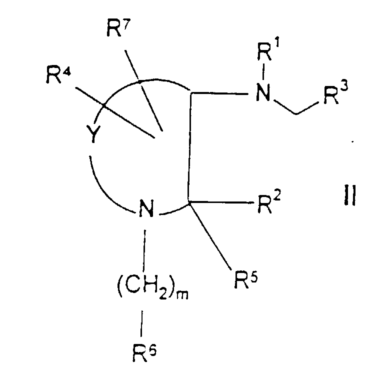
wherein Y is (CH₂)ₙ where n is an integer ranging from 1 to 6 and any one of the carbon-carbon single bonds in said (CH₂)ₙ may optionally be replaced by a carton-cartco double bond, and any one of the carbon atoms of said (CH₂)ₙ may optionaily be substituted with R⁴ or R⁷;
m is an integer ranging from 0 to 8, and any one of the carbon-carbon single bonds of said (CH₂)ₘ may optionally be replaced by a carbon-carbon double bond or a carbon-carbon triple bond ;
R¹ is hydrogen or (C₁-C₆)alkyl optionally substituted with hydroxy, alkoxy or fluoro ;
R² is selected from hydrogen, (C₁-C₆)alkyl, (C₃-C₇)cycoalk, wherein one of the carbon atoms of said cycloalkyl may optionally be replaced by nitrogen, oxygen or sulfur, aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl ; phenyl-(C₂-C₆)alkyl, benzhydryl and benzyl, wherein each of said aryl and heteroaryl groups and the phenyl moieties of said benzyl, phenyl-(C₂-C₆)alkyl and benzhydryl may optionally be substituted with 1 to 3 substituents independently selected from halo, nitro. (C₁-C₆)alkyl, (C₁-C₆)alkoxy, trifluoromethyl, amino, (C₁-C₆)alkylamino, (C₁-C₆)alkyl-C-(O)-O-, (C₁-C₆)alkyl-O-C(O)-, (C₁-C₆)alkyl, (C₁-C₆)alkyl-C(O)-O-, (C₁-C₆)alkyl-C(O)-(C₁-C₆)alkyl-O-, (C₁-C₆)alkyl-C(O)-, (C₁-C₆)alkyl-C(O)-(C₁-C₆)alkyl-, di-(C₁-C₆)alkylamino, -C(O)-NH-(C₁-C₆)alkyl, (C₁-C₆)alkyl-C(O)-NH-(C₁-C₆)alkyl, -NHC(O)H and -NHC(O)-(C₁-C₆)alkyl; and wherein one of the phenyl moieties of said benzhydryl may optionally be replaced by naphthyl, thienyl. furyl or pyridyl ;
R⁵ is hydrogen, phenyl or (C₁-C₆)alkyl ;
or R² and R⁵, together with the carbon to which they are attached, form a saturated carbocyclic ring having from 3 to 7 carbon atoms wherein one of said carbon atoms may optionally be replaced by oxygen, nitrogen or sulfur,
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl ; and cycloalkyl having 3 to 7 carbon atoms wherein one of said carbon atoms may optionally be replaced by nitrogen, oxygen or sulfur; wherein each of said aryl and heteroaryl groups may optionally be substituted with 1 to 3 substituents, and said (C₃-C₇)cycloalkyl may optionally be substituted with one or two substituents, each of said substituents being independently selected from halo, nitro, (C₁-C₆)alkyl, (C₁-C₆)alkoxy, methyl, trifluoromethyl, trifluoromethoxy, phenyl, amino, (C₁-C₆)alkylamino, -C(O)-NH-(C₁-C₆)alkyl, (C₁-C₆)alkyl-C(O)-NH-(C₁-C₆)alkyl, -NHC(O)H and -NHC(O)-(C₁-C₆)alkyl; and
R⁴ and R⁷ are each independently selected from hydrogen, hydroxy, halo, amino, oxo (=O), nitrile, (C₁-C₆)alkylamino, di-(C₁-C₆)alkylamino, (C₁-C₆)alkoxy, (C₁-C₆)alkyl-O-C(O)-, (C₁-C₆)alkyl-O-C(O)-(C₁-C₆)alkyl-, hydroxy-(C₁-C₆)alkyl, (C₁-C₆)alkoxy-(C₁-C₆)alkyl, (C₁-C₆)alkyl-C(O)-O-, (C₁-C₆)alkyl-C(O)-(C₁-C₆)alkyl-O-, (C₁-C₆)alkyl-C(O)-, (C₁-C₆)alkyl-C(O)-(C₁-C₆)alkyl-, and the radicals set forth in the definition of R² ;
R⁶ is hydrogen, NHC(O)R⁸, NHCH₂R⁸, SO₂R⁸ or one of the radicals set forth in the definitions of R², R⁴ and R⁷; and,
R⁸ is (C₁-C₆)alkyl, hydrogen, phenyl, or phenyl-(C₁-C₆)alkyl;
or a pharmaceutically acceptable salt of such compound: or
(C) of a compound of the formula 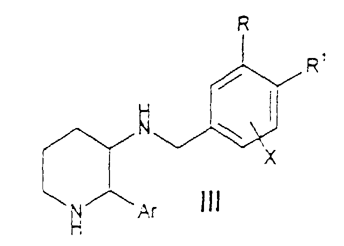 wherein
R is C₁-C₈ alkoxy, C₁-C₆ alkyl substituted by halo, C₂-C₆, alkenyl substituted by halo C₂-C₆ alkynyl substituted by halo, or C₁-C₆, alkyl substituted by halo and hydroxy;
R¹ is H, halo, or C₁-C₆ alkoxy;
or R and R¹ are taken together with the two carbons to which they are attached to form a fused C₄-C₅ cycloalkyl ring wherein one carbon atom is optionally replaced by oxygen and wherein one or two carbon carbon atoms are optionally substituted by 1 to 5 substituents independently selected from halo, C₁-C₆ alkyl and C₁-C₆ alkyl substituted by halo;
X is C₁-C₆ alkoxy, C₁-C₆ alkoxy substituted by halo, phenoxy or halo; and
Ar is phenyl optionally substituted by halo;
or a pharmaceutically acceptable salt of such compound, for the manufacture of a medicament for the curative or prophylactic treatment of the symptoms of irritable bowel syndrome.

2. The use according to claim 1 wherein the symptom that is treated is abdominal pain associated with irritable bowel syndrome.

3. The use according to claim 1 or claim 2 wherein the compound is selected from the group consisting of
(2S, 3S)-2-diphenylmethyl-3-(5-tert-butyl-2-methoxybenzyl)amino-1-azabicyclo[2.2.2]octane,
(2S, 3S)-3-(2-methoxybenzyl)amino-2-phenyl-piperidine,
(2S, 3S)-3-(5-trifluoromethoxy-2-methoxybenyl)amino-2-phenyl-piperidine,
(2S, 3S)-2-diphenylmethyl-3-(5-isopropyl-2-methoxybenzyl)amino- 1-azabicyclo[2.2,2]octane,
(2S, 3S)-2-diphenylmethyl-3(2-methoxybenzyl)amino-1-azabicyclo[2,2.2]octane,
(3R,4S,5S,6S)-5-(5-isopropyl-2-methoxybenzyl)amino-6-diphenylmethyl- 1-azabicyclo[2. 2.2]octane-3-carboxylic acid,
and the pharmaceutically acceptable salts of the foregoing compounds.

4. The use according to claim 3 wherein the compound is (2S,3S)-2-diphenylmethyl-3-(5-tert-butyl-2-methoxybenzyl)amino-1-azabicyclo[2.2.2]octane or a pharmaceutically acceptable salt of said compound.

5. The use of a NK-1 receptor antagonist, or a pharmaceutically acceptable salt thereof, for the manufacture of a medicament for the curative or prophylactic treatment of the symptoms of irritable bowel syndrome.

6. The use according to claim 5 wherein said NK-1 receptor antagonist is a substance P receptor antagonist.

7. The use according to claim 5 or claim 6 wherein the symptom that is treated is abdominal pain associated with irritable bowel syndrome.
